# EUROPEAN PATENT APPLICATION

(11) **EP 2 052 687 A1**
(43) Date of publication of application: **29.04.2009**
(21) Application number: 07792153.4
(22) Date of filing: 08.08.2007
(51) Int. Cl.: A61B 17/00, A61M 37/00, B05B 7/00

(54) **TOOL AND DEVICE FOR USE IN SCLEROTHERAPY FOR VARICOSE VEIN**

(30) Priority: 09.08.2006 JP 2006216804
(71) Applicant: JMS Co., Ltd., Hiroshima-shi, Hiroshima 730-8652 (JP)
(72) Inventor: SHIMIZU, Yasuhiro, Hiroshima-shi Hiroshima 732-0817 (JP); HAYASHI, Shuro, Hiroshima-shi Hiroshima 730-8652 (JP)
(74) Representative: Collin, Jérôme
(86) International application number: PCT/JP2007/065485
(87) International publication number: WO 2008/018484

(57) **Abstract**

A mixing portion is provided between a medical fluid container connecting portion 5 to which an opening of a medical fluid container 100 for a sclerosing agent is to be connected and a gas container connecting portion 6 to which an opening of a gas container 200 for a gas is to be connected. The mixing portion has a communication passageway R for communication between the openings of the containers 100 and 200 connected to the connecting portions 5 and 6. The mixing portion is deformable such that relative positions of the connecting portions 5 and 6 can be changed.

## Description

### Technical Field

The present invention relates to an instrument and device for sclerotherapy of varix formed in, for example, a leg vein.

### Background Art

In general, for example, when a varix is formed in a vein present in a leg of an organism, the skin surface has an elevation corresponding to the shape of the varix or has a livid area, which is cosmetically undesirable. If such symptoms of varix are further exacerbated, intractable dermatitis or skin necrosis can occur. In any of these cases, treatments less invasive to patients are desirable.

Treatment of varix includes a variety of approaches, which are designed depending on the type of a varicose vein, symptoms, etc. Presently, such approaches are appropriately adopted, singly or in combination, for varicose therapy. Among a variety of treatments, sclerotherapy is adopted in many cases for therapy of a varix formed in a relatively thin vein, such as reticular varicose veins, spider-web varicose veins, small saphenous varicose veins, etc. Sclerotherapy includes injecting a sclerosing agent into part of a vein closer to the center of the vasculature than a varix or directly into a varicose vein to damage the intravascular wall such that inflammation of the intravascular wall is caused. Thereby, thrombosis and adhesion of the intravascular wall are caused to obliterate the vein and block the blood flow, so that shrinkage of the vein occurs (see, for example, Non-patent Documents 1 and 2 identified below).

Non-patent Document 1 discloses using Polidocanol, which is a type of surfactant, as the sclerosing agent to lessen the pain a patient has during injection of the sclerosing agent into the vein and to prevent skin pigmentation after the treatment. Non-patent Document 1 describes that Polidocanol is foamed when used and that the instruments used for foaming are a medical fluid syringe filled with liquid Polidocanol, an air syringe filled with air, and a T-shape stopcock. The both syringes are made of common plastic materials. The T-shape stopcock is one that is commonly used in medical workplaces, which has three connecting portions integrally arranged orthogonal to one another in a plane.

When foaming Polidocanol, luer-tapered portions of the medical fluid syringe and the air syringe are respectively connected with connecting portions of the T-shape stopcock, and valves of the T-shape stopcock are in such a state that the two syringes are in communication. The operator thrusts the rod of the medical fluid syringe such that Polidocanol is flowed through the passageways of the T-shape stopcock. Then, the rod of the air syringe is strongly thrust into the syringe such that Polidocanol and air are intensely expelled out of the air syringe and flowed through the passageways of the T-shape stopcock. Polidocanol and air are intensely flowed through the passageways of the T-shape stopcock so as to mix with each other. As a result, Polidocanol is foamed, and the Polidocanol foam is flowed into the medical fluid syringe. Then, conversely, the rod of the medical fluid syringe is strongly thrust into the syringe such that the Polidocanol foam is flowed through the passageways of the T-shape stopcock. Thereby, the Polidocanol foam is transformed into finer foam, which is then flowed into the air syringe. This procedure is repeated to make the Polidocanol foam finer and finer.

To inject the thus-produced Polidocanol foam into a vein of a patient, a winged needle is put into the vein while the patient is kept standing upright. The patient is then moved into a dorsal position with legs raised, and Polidocanol is injected into the vein using a syringe via the winged needle. After the injection of Polidocanol, the injection point in the legs is strongly pressed by elastic bandage, elastic socks, elastic stocking, or the like.

Injection of such air-mixed Polidocanol foam into a vein enables Polidocanol to spread throughout the vein even with a smaller quantity of Polidocanol injected as compared with injection of liquid Polidocanol, and also secures a longer time for Polidocanol to be in contact with the intravascular wall because the spread Polidocanol foam flows very unsmoothly in the vein and therefore has a longer residence time.

Thus, the intravascular wall of the vein can be damaged without fail using a smaller quantity of Polidocanol. As a result, the probability of shock symptoms during surgical operation, allergic symptoms, bronchospasm, acute complications, such as drug intoxication, or the like, due to Polidocanol decreases such that less invasive varicose sclerotherapy can be carried out.
[Non-patent Document 1] Takaaki ITO, Diagnosis and Therapy of Leg Varices, Monthly Book Derma No. 89, Kabushiki Kaisha ZEN NIHON BYOIN SHUPPANKAI, July 30, 2004, p. 18, p. 19, p. 47, p. 48.
[Non-patent Document 2] Takehisa IWAI and 5 others, Current Affairs of Leg Varicose Sclerotherapy, IGAKU-SHOIN Ltd., November 1, 1997, pp. 2-3.

### Disclosure of Invention

### Problems to be solved by the invention

Injection of Polidocanol foam into a vein as disclosed in Non-patent Document 1 requires Polidocanol to be in contact with the largest possible area of the intravascular wall and also requires the longest possible residence time for Polidocanol, and therefore, finer foam is demanded. Thus, to produce Polidocanol foam, the operator needs to continuously repeat the aforementioned process of alternately moving the rod of the medical fluid syringe and the rod of air syringe.

However, Non-patent Document 1 discloses that the T-shape stopcock having three connecting portions fixed such that they are orthogonal to one another in a plane is respectively connected to the medical fluid syringe and to the air syringe. Accordingly, the two syringes are orthogonal, so that the medical fluid syringe with its rod and the air syringe with its rod are apart from each other. With such a structure, the operator has difficulty in manipulating the two rods of the syringes because the operator needs to spread the arms wider than comfortable positions with the hands holding the syringes and the rods, and this position makes it difficult for the operator to strongly thrust the rods. Also, it is difficult for the operator to repeatedly move the two rods because the reciprocating directions of the rods are orthogonal to each other, resulting in inefficient workability.

The present invention was conceived in view of the above circumstances, with an objective of improving the workability in the operation of mixing a gas into a sclerosing agent used in varicose sclerotherapy to produce foam of the sclerosing agent.

### Means for solving the problems

To achieve the above objective, in the first invention, when mixing a sclerosing agent and a gas in a mixing portion having a communication passageway for communication between an opening of a medical fluid container for the sclerosing agent and an opening of a gas container for the gas, the mixing portion is deformed to change the relative positions of the containers.

Specifically, the instrument includes: a medical fluid container connecting portion to which an opening of a medical fluid container for a sclerosing agent is to be connected; a gas container connecting portion to which an opening of a gas container for a gas is to be connected; and a mixing portion provided between the connecting portions, the mixing portion having a communication passageway for communication between the openings of the containers connected to the connecting portions, the communication passageway allowing mixture of the sclerosing agent and the air flowing therethrough, wherein the mixing portion is deformable such that relative positions of the connecting portions can be changed.

In this structure, when an operator manipulates the medical fluid container such that the sclerosing agent is flowed through the communication passageway of the mixing portion into the gas container and then manipulates the gas container such that the sclerosing agent and the gas are flowed through the communication passageway, the sclerosing agent and the gas are mixed in the communication passageway to produce foam of the sclerosing agent, which is then flowed into the medical fluid container. This procedure is repeated so that the sclerosing agent is transformed into finer foam. In the manipulation of the two containers, the mixing portion can be deformed to change the relative positions of the medical fluid container connecting portion and the gas container connecting portion. Therefore, the medical fluid container and the gas container can be positioned in view of easier manipulation for the operator. The procedure for foaming the sclerosing agent may be started with manipulation of the gas container such that the gas is first flowed into the medical fluid container.

According to the second invention, in the structure of the first invention, the mixing portion is deformable such that the medical fluid container and the gas container are positioned side by side.

In this structure, the medical fluid container and the gas container can be positioned side by side in view of easier manipulation for the operator.

According to the third invention, in the structure of the first or second invention, the mixing portion has a narrow part in which the communication passageway is narrower.

In this structure, the flow rate of the sclerosing agent and the gas flowing through the communication passageway increases when they flow through the narrow part, so that mixing of the sclerosing agent and the gas is enhanced.

According to the fourth invention, in the structure of any one of the first to third inventions, the communication passageway has a projection elevated toward the inside of the communication passageway.

In this structure, part of the communication passageway is narrower due to the projection, so that mixing of the sclerosing agent and the gas is enhanced as in the third invention.

According to the fifth invention, in the structure of any one of the first to fourth inventions, the mixing portion is made of a resin.

In this structure, the mixing portion can easily be deformed to change the relative positions of the connecting portions.

According to the sixth invention, in the structure of the fifth invention, the mixing portion has a tubular part and is deformable such that the tubular part is bent.

In this structure, the communication passageway is narrower at a bent portion of the tubular part, so that mixing of the sclerosing agent and the gas is enhanced as in the third invention.

According to the seventh invention, in the structure of any one of the first to sixth inventions, the medical fluid container and the gas container are syringes; and the medical fluid container connecting portion and the gas container connecting portion are designed such that tapered surfaces provided in external surfaces of the syringes at the tip ends fit in the connecting portions.

In this structure, in the manipulation of the rods of the medical fluid syringe and the gas syringe, the tip end portions of the syringes can be prevented from unexpectedly falling off the connecting portions. After the sclerosing agent foam is produced, the sclerosing agent is flowed into one of the syringes and then the tip end of the syringe is disconnected from the connecting portion, so that the sclerosing agent can be injected into a vein using this syringe.

According to the eighth invention, in the structure of any one of the first to seventh inventions, at least one of the medical fluid container and the gas container is a syringe with a rod inserted therein; and the syringe is provided with an urging member for urging the rod in such a direction that the rod is extracted from the syringe.

In this structure, when the operator thrusts the rod into the syringe and takes the hand off the rod, the rod moves in such a direction that the rod is extracted from the cylinder by the urging member.

According to the ninth invention, the urging member is a spring.

According to the tenth invention, in the structure of any one of the first to sixth inventions, the medical fluid container and the gas container are bags.

In this structure, when one of the bags is squeezed, the sclerosing agent and/or the gas are flowed into the other bag.

According to the eleventh invention, in the structure of the tenth invention, an opening of at least one of the medical fluid container and the gas container is detachably connected to the connecting portion.

In this structure, after the sclerosing agent foam is produced, the sclerosing agent is flowed into one of the bags and then the bag is disconnected from the connecting portion, so that the sclerosing agent can be injected into a vein.

The twelfth invention is a device for varicose sclerotherapy, including: a medical fluid container containing a sclerosing agent; a gas container containing a gas; and an instrument connecting the medical fluid container and the gas container, wherein the instrument includes a medical fluid container connecting portion to which an opening of the medical fluid container is connected, a gas container connecting portion to which an opening of the gas container is connected, and a mixing portion provided between the connecting portions, the mixing portion having a communication passageway for communication between the openings of the containers connected to the connecting portions, the communication passageway allowing mixture of the sclerosing agent and the air flowing therethrough, and the mixing portion is deformable such that relative positions of the connecting portions can be changed.

In this structure, the medical fluid container and the gas container can be positioned in view of easier manipulation for the operator for foaming the sclerosing agent as in the first invention.

### Effects of the invention

According to the first invention, the mixing portion in which the sclerosing agent and the gas are mixed is deformed such that the relative positions of the connecting portions can be changed. Therefore, the medical fluid container and the gas container can be positioned in view of easier manipulation for the operator. Thus, the workability in the operation of mixing the gas into the sclerosing agent to produce foam of the sclerosing agent is improved.

According to the second invention, the medical fluid container and the gas container can be positioned side by side in view of easier manipulation for the operator. Thus, the workability can be further improved.

According to the third invention, mixing of the sclerosing agent and the gas can be enhanced by the partially narrowed communication passageway, and the time for producing extremely fine foam of the sclerosing agent can be shortened.

According to the fourth invention, the time for producing extremely fine foam of the sclerosing agent can be shortened as in the third invention.

According to the fifth invention, the mixing portion is made of a resin. Therefore, the containers can readily be positioned in view of easier manipulation.

According to the sixth invention, the mixing portion is deformable such that the tubular part is bent. Therefore, a narrower part can be formed in the communication passageway only by positioning the containers in view of easier manipulation, and the time for producing extremely fine foam of the sclerosing agent can be shortened as in the third invention.

According to the seventh invention, the tip ends of the syringes are fit in the medical fluid container connecting portion and the gas container connecting portion. Therefore, the syringes can be prevented from unexpectedly falling off the connecting portions, so that the handleability is further improved, and the produced sclerosing agent foam can be injected into a vein using the syringe.

According to the eighth and ninth inventions, the rod is extracted from the syringe only by taking the hand off the thrust rod, and therefore, the manipulation of the rod is easy.

According to the tenth invention, the medical fluid container and the gas container are bags, so that the sclerosing agent and the gas can readily be flowed out of the containers, and the handleability can further be improved.

According to the eleventh invention, the bag containing the sclerosing agent foam can be disconnected from the connecting portion, and this bag can be used for injection of the sclerosing agent foam into a vein.

According to the twelfth invention, the workability in the operation of mixing the gas into the sclerosing agent to produce foam of the sclerosing agent can be further improved as in the first invention.

### Brief Description of Drawings

FIG. 1 is a side view of an instrument according to an embodiment of the present invention.
FIG. 2 illustrates the instrument connected with a medical fluid syringe and an air syringe, which corresponds to FIG. 1.
FIG. 3 illustrates the state of the instrument connected with the syringes when used.
FIG. 4 is a cross-sectional view of the instrument of FIG. 2.
FIG. 5 illustrates how to use the instrument.
FIG. 6 illustrates the first variation of the embodiment, which corresponds to FIG. 2.
FIG. 7 illustrates the first variation of the embodiment, which corresponds to FIG. 3.
FIG. 8 illustrates the second variation of the embodiment, which corresponds to FIG. 1.
FIG. 9 illustrates the second variation of the embodiment, which corresponds to FIG. 4.
FIG. 10 illustrates the third variation of the embodiment, which corresponds to FIG. 1.
FIG. 11 illustrates the fourth variation of the embodiment, which corresponds to FIG. 1.
FIG. 12 illustrates the fifth variation of the embodiment, which corresponds to FIG. 3.
FIG. 13 illustrates the sixth variation of the embodiment, which corresponds to FIG. 2.
FIG. 14 illustrates the sixth variation of the embodiment, which corresponds to FIG. 3.
FIG. 15 illustrates the seventh variation of the embodiment, which corresponds to FIG. 1.
FIG. 16 illustrates the eighth variation of the embodiment, which corresponds to FIG. 2.
FIG. 17 is a cross-sectional view of the instrument of FIG. 16 taken along line A-A.
FIG. 18 is a side view of a winged needle for injection of a sclerosing agent.

### Description of Reference Numerals

- 1: instrument
- 2: circular tube (tubular portion)
- 3: medical fluid side cylindrical member
- 3b: greater diameter cave
- 4: air side cylindrical member
- 4b: greater diameter cave
- 5: medical fluid container connecting portion
- 6: gas container connecting portion
- 20: narrow part
- 100: medical fluid syringe (medical fluid container)
- 101a: opening
- 202: piston
- 103: rod
- 200: air syringe (gas container)
- 201a: opening
- 202: piston
- 203: rod
- 150: medical fluid bag (medical fluid container)
- 210: coil spring (urging member)
- 250: air bag (gas container)
- R: communication passageway

### Best Mode for Carrying Out the Invention

Hereinafter, an embodiment of the present invention is described with reference to the drawings. It should be noted that the descriptions of the preferable embodiment below are merely exemplary in essence and do not intend to limit the present invention or the applications or uses thereof.

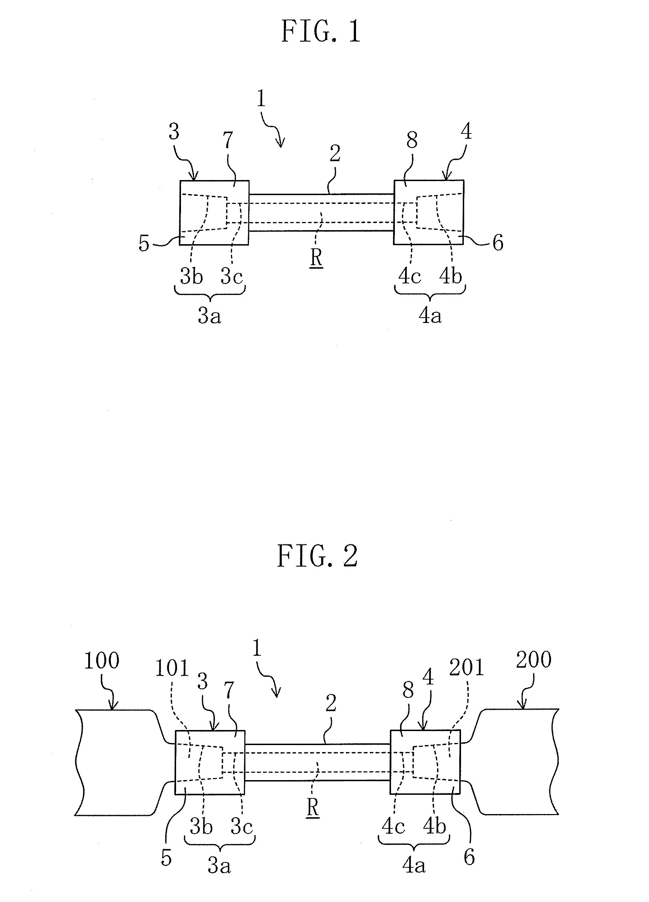

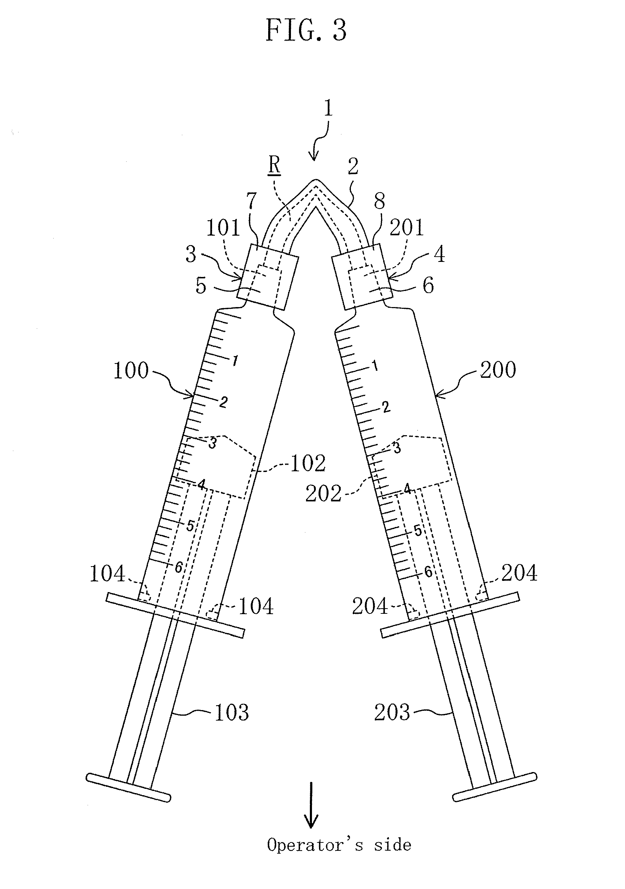

FIG. 1 shows an instrument 1 for use in varicose sclerotherapy according to an embodiment of the present invention. The instrument 1 is connectable with a medical fluid syringe 100 containing a sclerosing agent and an air syringe 200 containing air as shown in FIGS. 2 and 3 and is designed such that the sclerosing agent and the air from the syringes 100 and 200 are mixed to produce foam of the sclerosing agent. The instrument 1 is used for a therapy in which such sclerosing agent foam is injected into a vein, so-called foam sclerotherapy.

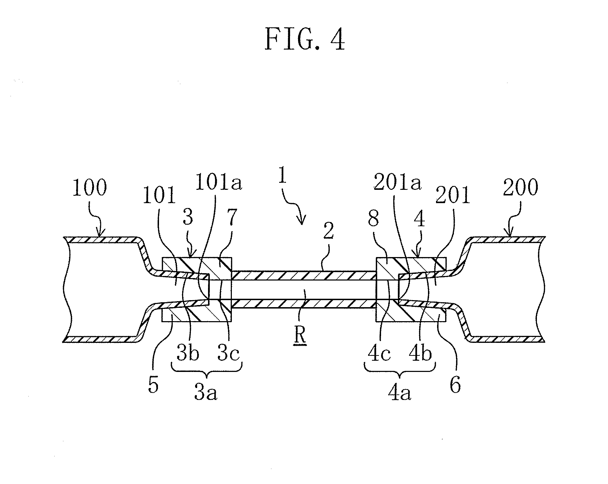

It should be noted that, as shown in FIG. 3, the medical fluid syringe 100 and the air syringe 200 are of disposable type commonly used in medical workplaces, which are made of transparent resin, and respectively correspond to a medical fluid container and a gas container of the present invention. The syringes 100 and 200 have luer-tapered portions 101 and 201, respectively. The outer surfaces of the luer-tapered portions 101 and 201 are each tapered off to the end with a gradient of 6/100. The end faces of the luer-tapered portions 101 and 201 have openings 101a and 201 a, respectively, as shown in FIG. 4. The gradient of the tapered surfaces can arbitrarily be determined. Numerals 102 and 202 denote pistons, and numerals 103 and 203 denote rods. The tip ends of the rods 103 and 203 are stuck in the pistons 102 and 202, respectively, such that the pistons 102 and 202 and the rods 103 and 203 are readily separable. The inner surfaces of the medical fluid syringe 100 and the air syringe 200 have stoppers 104 and 204 at the basal end for preventing the pistons 102 and 202 from falling off the syringes 100 and 200. The stoppers 104 and 204 may be annular around the whole inner perimeter of the syringes 100 and 200 or may be intermittently provided around the inner perimeter of the syringes.

The instrument 1 includes a resin circular tube 2, which corresponds to a tubular portion of the present invention, a medical fluid side cylindrical member 3 provided at one end of the circular tube 2, and an air side cylindrical member 4 provided at the other end of the circular tube 2. The resin material of the circular tube 2 is not limited to any particular material so long as it has such flexibility that the circular tube 2 is bendable by the force from an operator. Examples of the resin material include silicones and vinyl chlorides. These resin materials may preferably have translucency. The circular tube 2 has substantially the same diameter and the same cross-sectional shape between its ends and is generally linear when no external force applied. When external force is applied to the circular tube 2 such that the circular tube 2 is bent, part of the circular tube 2 at the point of bending is deformed.

The medical fluid side cylindrical member 3 is made of a resin material harder than the resin material used for the circular tube 2. The outside diameter of the medical fluid side cylindrical member 3 is greater than that of the circular tube 2. Referring to FIG. 4, the central part of the medical fluid side cylindrical member 3 has a through hole 3a extending along the center line of the cylindrical member 3. The cylindrical member 3 has a greater diameter cave 3b at the end of the hole 3a opposite to the circular tube 2, which is greater than the inside diameter of the circular tube 2, and a smaller diameter cave 3c at the other end closer to the circular tube 2, which is substantially equal to the inside diameter of the circular tube 2. The greater diameter cave 3b is shaped such that the tapered surface of the luer-tapered portion 101 fits in the cave 3b. The air side cylindrical member 4 has substantially the same structure as the medical fluid side cylindrical member 3 and has a through hole 4a with a greater diameter cave 4b and a smaller diameter cave 4c.

One end of the circular tube 2 is fixed to an end face of the medical fluid side cylindrical member 3, and the other end is fixed to an end face of the air side cylindrical member 4, such that the circular tube 2, the medical fluid side cylindrical member 3 and the air side cylindrical member 4 are integrated. In this state, the hole 3a of the medical fluid side cylindrical member 3 and the hole 4a of the air side cylindrical member 4 are in communication with each other via the hollow of the circular tube 2. The interval between the circular tube 2 and the medical fluid side cylindrical member 3 and the interval between the circular tube 2 and the air side cylindrical member 4 are sealed such that air-tightness is secured. When the members 2 to 4 are integrated, the flexibility of the circular tube 2 enables the operator to change the relative positions of the cylindrical members 3 and 4 by grasping and moving the cylindrical members 3 and 4, for example.

Part of the medical fluid side cylindrical member 3 corresponding to the greater diameter cave 3b constitutes a medical fluid container connecting portion 5, and part of the air side cylindrical member 4 corresponding to the greater diameter cave 4b constitutes an gas container connecting portion 6. Part of the medical fluid side cylindrical member 3 corresponding to the smaller diameter cave 3c (portion 7), part of the air side cylindrical member 4 corresponding to the smaller diameter cave 4c (portion 8), and the circular tube 2 constitute a mixing portion of the present invention. The smaller diameter cave 3c of the hole 3, the smaller diameter cave 4c of the hole 4 and the hollow of the circular tube 2 constitute a communication passageway R of the present invention.

How to foam the sclerosing agent using the instrument 1 which has the above structure is now described. The sclerosing agent used herein is Polidocanol, which is a type of surfactant. The concentration of Polidocanol and the amount of Polidocanol to be injected may be adjusted depending on, for example, the physical properties of a patient, the size and area of a varix, or the site of the patient where the varix exists. For example, the concentration of Polidocanol is from 1% to 3%. The amount of Polidocanol to be injected is equal to or less than the safety dose limit. In the case where 3% Polidocanol is injected to a patient of 60 kg, the amount of Polidocanol may be 4 ml or less. In this embodiment, 3 ml of 1% Polidocanol is contained in the medical fluid syringe 100, and 3 ml of air is contained in the air syringe 200. The mixing ratio of Polidocanol and air can be adjusted depending on, for example, the size of a varix.

The luer-tapered portion 101 of the medical fluid syringe 100 is inserted into the greater diameter cave 3b, and the luer-tapered portion 201 of the air syringe 200 is inserted into the greater diameter cave 4b, as shown in FIG. 2, whereby the medical fluid syringe 100 and the air syringe 200 are retained by the instrument 2. With the elements thus arranged, the operator grasps the two syringes 100 and 200 in different hands to bend the circular tube 2, as shown in FIG. 3, such that the operator can easily perform the operation. Specifically, the syringes 100 and 200 are held side by side such that the rod insertion side of the two syringes 100 and 200 is on the operator side and that the angle between the center lines of the syringes 100 and 200 is from about 20° to about 40°. In this process, the operator can readily bend the circular tube 2 even with small force due to its flexibility to easily change the positions of the syringes 100 and 200. While the circular tube 2 is kept bent, the communication passageway R is narrowed at the bending point.

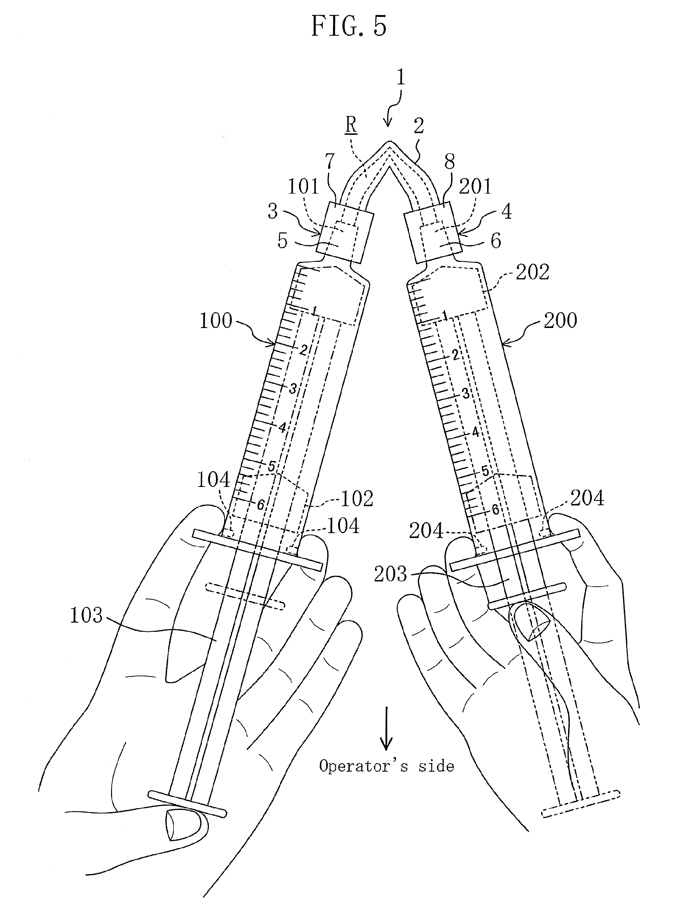

Then, as shown in FIG. 5, the rod 103 is thrust into the medical fluid syringe 100 such that the sclerosing agent flows into the air syringe 200 via the communication passageway R. Then, the rod 203 is strongly thrust into the air syringe 200 such that the sclerosing agent and the air flow through the communication passageway R. Strongly thrusting the rod 203 into the air syringe 200 increases the flow rate of the sclerosing agent and the air, which are mixed together in the communication passageway R and transformed into foam. The resultant foam of the sclerosing agent is flowed into the medical fluid syringe 100. The communication passageway R has narrowed part, at which the flow rate of the sclerosing agent and the air further increases so that mixing of these components is enhanced.

Then, the rod 103 is strongly thrust into the medical fluid syringe 100 such that the sclerosing agent foam flows through the communication passageway R. Thereby, the sclerosing agent foam becomes finer. The resultant finer sclerosing agent foam is flowed into the air syringe 200. This procedure is repeated 10 times or more, so that extremely fine foam of the sclerosing agent is produced. Even while the operator is moving the rod 103 or 203, the operator can change the positions of the syringes 100 and 200 and, namely, can enjoy improved handleability throughout the operation.

The circular tube 2 has translucency and, therefore, the sclerosing agent flowing through the circular tube 2 can be observed by eyes. This enables the operator to immediately confirm during the operation how fine the sclerosing agent is foamed.

The process of foaming the sclerosing agent may be started with thrusting the rod 203 into the air syringe 200 such that the air is flowed into the medical fluid syringe 100. In this case, the resultant foam does not disappear in about 10 minutes.

After a certain period of time, the luer-tapered portion 101 of the syringe 100 is pulled out of the greater diameter cave 3b while the sclerosing agent is contained in the medical fluid syringe 100, whereby the syringe 100 is disengaged from the instrument 1.

On the other hand, although not shown, the patient is raised to stand upright for sticking a plurality of thin winged needles of about 23 G to 27 G, with intervals therebetween, into a vein to which the sclerosing agent is to be injected. Each of the winged needles is attached to a tube which is designed such that the luer-tapered portion 101 of the medical fluid syringe 100 fits in the tube.

The luer-tapered portion 101 of the syringe 100 charged with the sclerosing agent foam is connected with the tube of the winged needle for injecting the sclerosing agent into the vein. The injection speed of the sclerosing agent is adjusted depending on the conditions of the varix. Injection of the sclerosing agent foam through the respective winged needles enables the sclerosing agent to spread throughout the vein even with a smaller quantity of sclerosing agent injected as compared with injection of liquid sclerosing agent, and also secures a longer time for the sclerosing agent to be in contact with the intravascular wall because the spread sclerosing agent foam flows very unsmoothly in the vein and therefore has a longer residence time. Thus, the intravascular wall of the vein can be damaged without fail using a smaller quantity of the sclerosing agent. As a result, the probability of shock symptoms during surgical operation, allergic symptoms, bronchospasm, acute complications, such as drug intoxication, or the like, due to the sclerosing agent decreases, and also, the probability of postoperative complications, such as hyperpigmentation of the skin, thrombophlebitis, skin necrosis, etc., decreases. Thus, less invasive varicose sclerotherapy can be carried out.

Immediately after the injection of the sclerosing agent, the area in which the sclerosing agent is injected is compressedly wrapped with a thin autohesive elastic bandage.

As described above, in the instrument 1 for use in varicose sclerotherapy according to this embodiment, the operator can deform the circular tube 2 to hold the medical fluid syringe 100 and the air syringe 200 side by side such that the operator can easily handles the instrument 1. This improves the workability of the instrument 1 during the operation of mixing the air into the sclerosing agent to foam the sclerosing agent.

Since the circular tube 2 is made of a flexible resin material, the production cost of the instrument 1 can be reduced while the handleability of the syringes 100 and 200 is improved.

Since the luer-tapered portions 101 and 201 of the syringes 100 and 200 are fitted in the greater diameter caves 3b and 4b of the instrument 1, the syringes 100 and 200 can be prevented from unexpectedly falling off the greater diameter caves 3b and 4b, and therefore, the handleability can further be improved. Since the syringes 100 and 200 can be disengaged from the instrument 1, the sclerosing agent foam can be injected by the medical fluid syringe 100 into a vein, and the procedure of varicose sclerotherapy can be simplified.

If the T-shape stopcock is used to foam the sclerosing agent as disclosed in Non-patent Document 1, the valve can be opened such that the three passageways of the T-shape stopcock are all in communication at the same time. This is for the purpose of flowing sterilization gas into the three passageways at one time during the sterilization process in the manufacture in the factory. When the valve is opened such that the three passageways are all in communication, it is probable that the air and the sclerosing agent leak from one of the three passageways of the T-shape stopcock with which no syringe is connected. However, this embodiment advantageously has only two ports at which the syringes 100 and 200 are connectable, so that the valve structure is unnecessary, and leakage of air and the sclerosing agent can be prevented in advance.

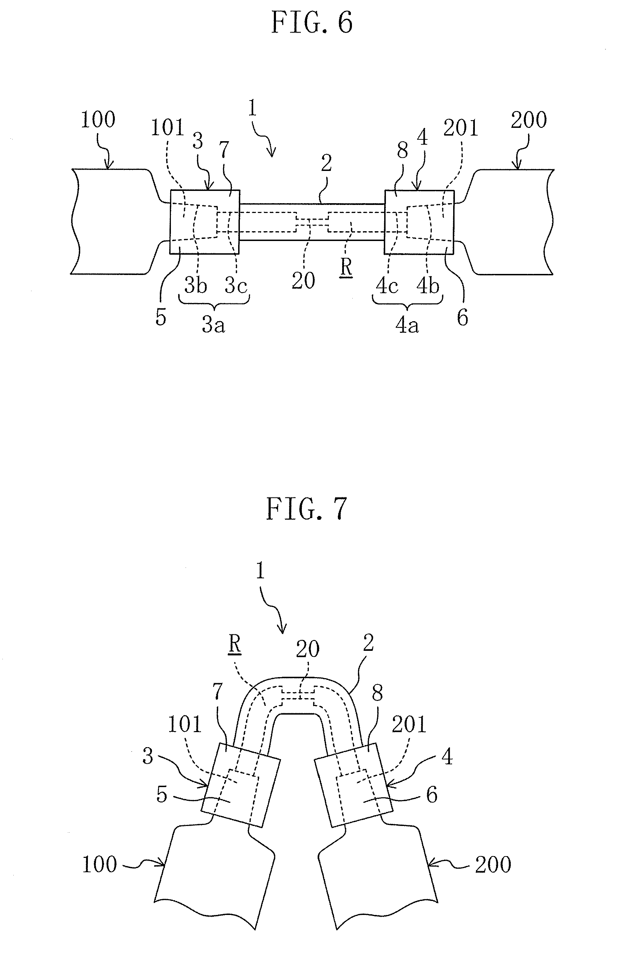

FIGS. 6 and 7 show the first variation of the above embodiment wherein the circular tube 2 may have a narrow part 20 at which the communication passageway R is constricted. This narrow part 20 is formed at the central part of the longitudinal dimension of the communication passageway R. The narrow part 20 is an annular projection in the inner wall of the communication passageway R, elevated toward the inside of the communication passageway R and continuing around the periphery of the communication passageway R. The narrow part 20 may be an integral part of the circular tube 2 or, alternatively, may be a part separable from the circular tube 2. In the first variation, the flow rate of the sclerosing agent and the air flowing through the communication passageway R further increases when passing through the narrow part 20, so that mixing of the sclerosing agent and the air is enhanced. As a result, finer foam of the sclerosing agent can be produced within a short period of time. The circular tube 2 has a thicker wall at the narrow part 20, and therefore, the narrow part 20 is kept generally straight as shown in FIG. 7 and the central part of the circular tube 2 does not deform when bent, so that the probability of completely closing the communication passageway R is avoided.

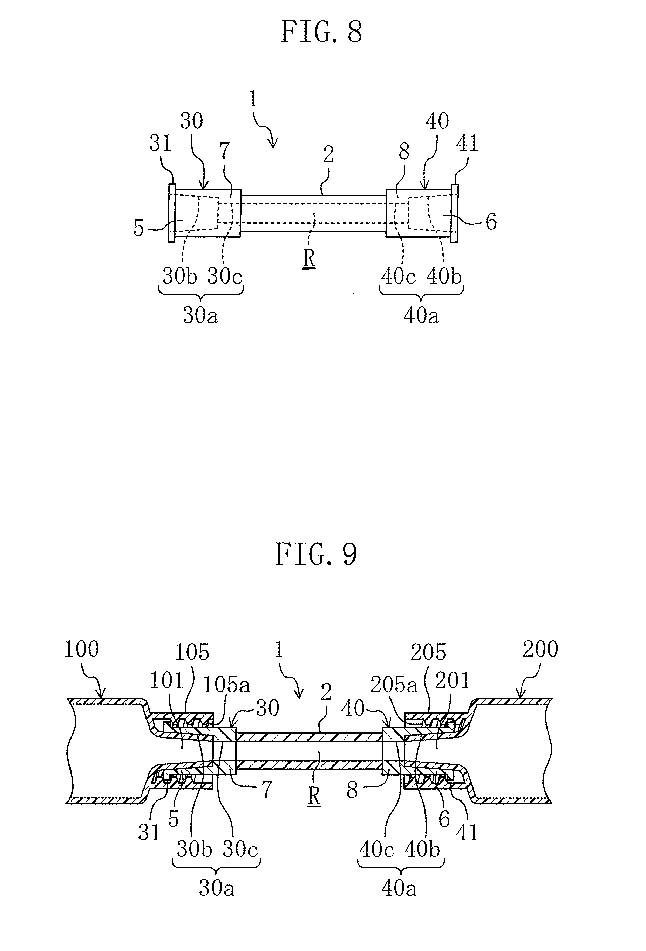

FIGS. 8 and 9 show the second variation wherein the syringes 100 and 200 have locking portions 105 and 205 at the tip ends. In this case, the medical fluid side cylindrical member 30 and the air side cylindrical member 40 of the instrument 1 may have engagement portions 31 and 41, respectively, for engagement with the locking portions 105 and 205. The locking portions 105 and 205 each have a cylindrical shape encompassing the luer-tapered portions 101 and 201. Provided between the locking portions 105 and 205 and the luer-tapered portions 101 and 201 are the spaces in which the cylindrical member 30 and 40 can be inserted. The inner surfaces of the locking portions 105 and 205 have external threads 105a and 205a, respectively. On the other hand, the engagement portion 31 of the medical fluid side cylindrical member 30 has ridges designed to establish screw engagement with the external thread 105a. The engagement portion 41 of the air side cylindrical member 40 also has similar ridges. Such screw engagement of the ridges 32 and 42 with the external threads 105a and 205a as shown in FIG. 9 enables the medical fluid syringe 100 and the air syringe 200 to be bound with the instrument 1, so that the syringes 100 and 200 can surely be prevented from unexpectedly falling off the instrument 1.

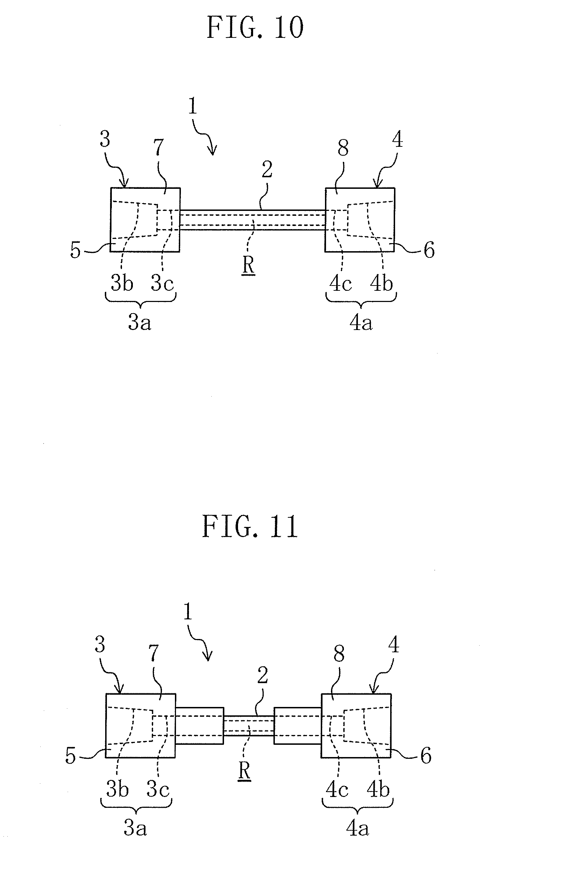

The instrument 1 may have, for example, the shape shown in FIG. 10 (third variation) or the shape shown in FIG. 11 (fourth variation). In the third variation, the outside diameter and inside diameter of the circular tube 2 are smaller than those of the first-described embodiment, such that the entire circular tube 2 constitutes the narrow part of the communication passageway R. In the fourth variation, the circular tube 2 is thinner in its intermediate part at around the center of the longitudinal dimension than at the longitudinal ends. This thinner part constitutes the narrow part of the communication passageway R.

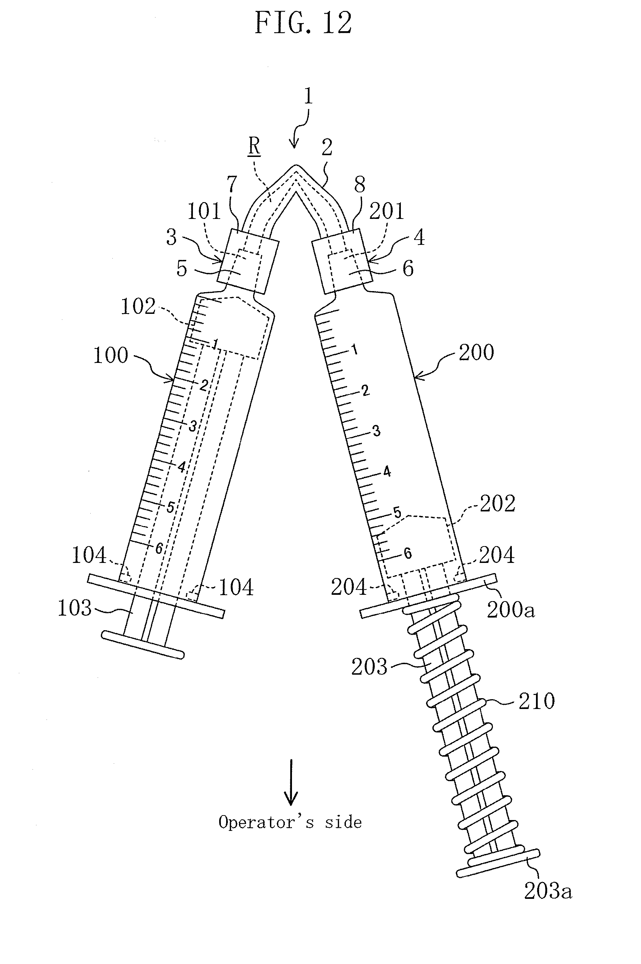

FIG. 12 shows the fifth variation wherein the air syringe 200 may be provided with a coil spring 210 (urging member) for urging the rod 203 in the extraction direction. The coil spring 210 has such an inside diameter that the rod 203 can be inserted into the coil spring 210. A longitudinal end of the coil spring 210 abuts on a planer stopper 203a provided at the basal end of the rod 203, and the other longitudinal end abuts on a flange 200a provided at the basal end of the syringe 200. Therefore, in the fifth variation, when the operator thrusts the rod 203 into the air syringe 200 against the urging force of the coil spring 210 and then reduces the thrusting force, the rod 203 is expelled by the coil spring 210 out of the air syringe 200. Accordingly, on the other hand, the rod 103 is drawn into the medical fluid syringe 100 because the syringe 100 is in communication with the air syringe 200. Namely, the operator repeats pushing and releasing the rod 203, whereby the sclerosing agent is alternately flowed into the medical fluid syringe 100 and the air syringe 200 so that the sclerosing agent can be foamed. The coil spring 210 may be provided to the medical fluid syringe 100 for urging the rod 103. Alternatively, the air syringe 200 may be provided with an urging member which urges the rod 203 in the opposite direction, i.e., toward the inside of the air syringe 200. The medical fluid syringe 100 may be provided with an urging member which urges the rod 103 toward the inside of the medical fluid syringe 100. The other examples of the urging member than the coil spring 210 include rubber, elastomer, etc.

In the case of the fifth variation, the resultant sclerosing agent foam is flowed into the medical fluid syringe 100 which does not have the coil spring 210 and injected into a vein from the syringe 100. Therefore, injection of the sclerosing agent is not interrupted by the urging force of the coil spring 210 and, thus, the amount of the sclerosing agent to be injected and the injection speed can readily be controlled.

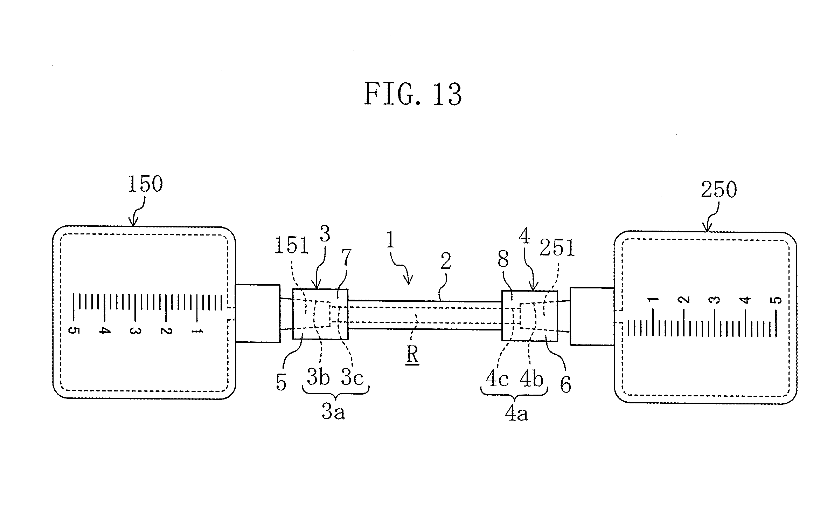

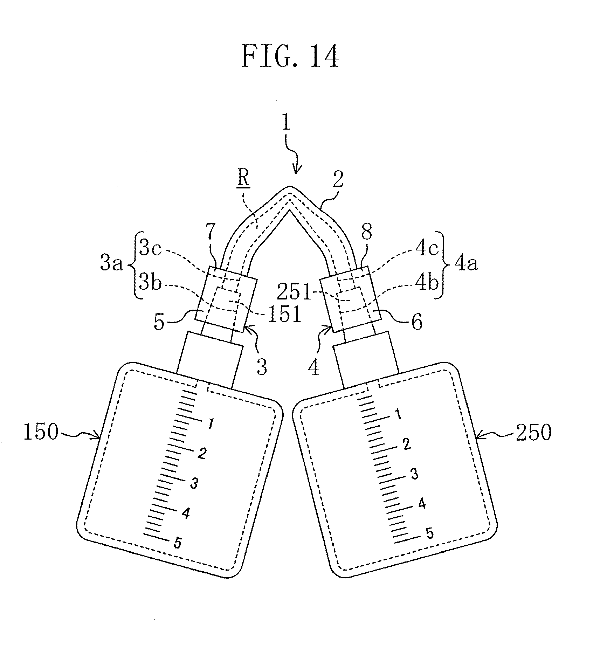

In this embodiment, the sclerosing agent and air are contained in the syringes 100 and 200 but may alternatively be contained in a medical fluid bag 150 (medical fluid container) and an air bag 250 (gas container), respectively, as shown in FIGS. 13 and 14 (sixth variation). The bags 150 and 250 are formed of a flexible resin film and have outlet portions 151 and 251 which are formed in the same way as the luer-tapered portions 101 and 201 of the syringes 100 and 200. The medical fluid bag 150 and the air bag 250 each have a printed scale on one surface, which is equivalent to that of the syringe.

The outlet portions 151 and 251 are inserted into the greater diameter caves 3b and 4b of the instrument 1, respectively, whereby the medical fluid bag 150 and the air bag 250 are fixed to the instrument 1. When the medical fluid bag 150 is squeezed, the sclerosing agent is flowed through the communication passageway R into the air bag 250. Then, when the air bag 250 is strongly squeezed, the sclerosing agent and the air are flowed through the communication passageway R and transformed into foam, which is then flowed into the medical fluid bag 150. Then, the medical fluid bag 150 is strongly squeezed so that the sclerosing agent foam becomes finer, and the finer foam is then flowed into the air bag 250. This procedure is repeated, so that extremely fine foam of the sclerosing agent can readily be produced.

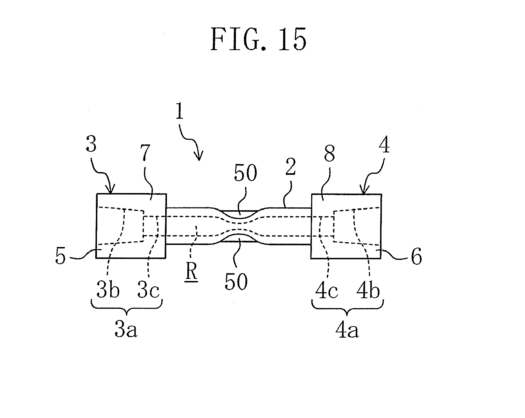

FIG. 15 shows the seventh variation wherein the circular tube 2 may have two weld portions 50 which are formed by applying pressure onto the longitudinal center of the circular tube 2 during external heat application. With these elements, a narrow part can be formed in the circular tube 2. The number of the weld portions 50 is not limited to two, but may be one or may be three or more.

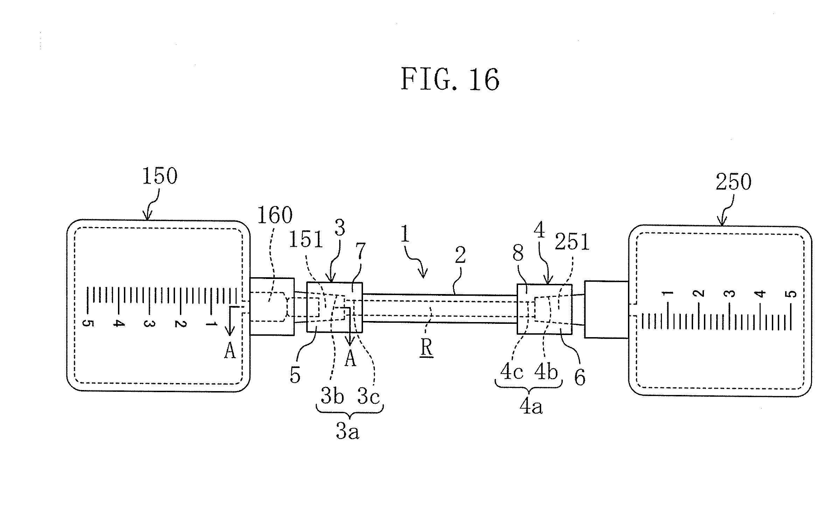

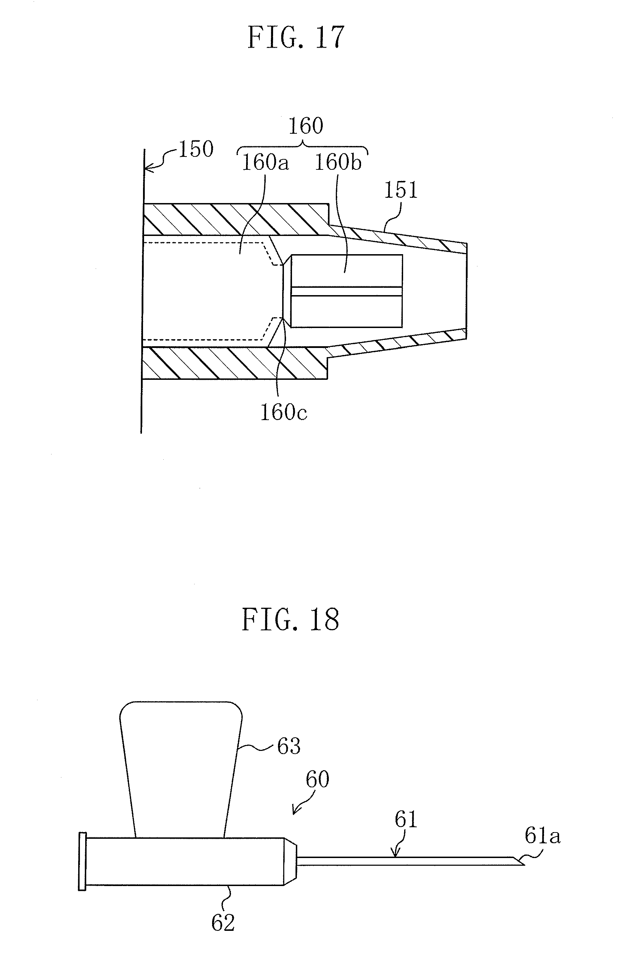

FIGS. 16 and 17 show the eighth variation wherein the outlet portion 151 of the medical fluid bag 150 may have a plug 160 for closing the inside passage of the outlet portion 151. As shown in FIG. 17, the inside of the outlet portion 151 has such a shape that its diameter decreases to the tip end in accordance with the luer-tapered shape of the external surface of the outlet portion 151. The plug 160 is formed by a cylindrical part 160a at the basal end of the outlet portion 151 and a separable portion 160b at the tip end of the outlet portion 151. The cylindrical part 160a and the separable portion 160b are formed of a hard resin as an integral part. The external surface of the cylindrical part 160a is fixed to the inner surface of the outlet portion 151. The plug 160 has a neck 160c between the cylindrical part 160a and the separable portion 160b. The diameter of the neck 160c is smaller than those of the cylindrical part 160a and the separable portion 160b. The neck 160c constitutes a fragile portion between the cylindrical part 160a and the separable portion 160b. The separable portion 160b has an elongated shape along the center line of the cylindrical part 160a, and a cross section of the separable portion 160b perpendicular to the center line substantially has the shape of a cross (+). The separable portion 160b closes the opening of the cylindrical part 160a at its end. The outside dimension of the separable portion 160b is greater than the inside dimension of the outlet opening of the outlet portion 151.

In the eighth variation, the path of the outlet portion 151 is closed by the separable portion 160b when the medical fluid is in stock. When the medical fluid is used, force is applied to the plug 160 from the outside of the outlet portion 151 to bend the plug 160 at the neck 160c. Thereby, the separable portion 160b is separated from the cylindrical part 160a so that the opening of the cylindrical part 160a at its end is opened. Accordingly, the medical fluid is discharged from the medical fluid bag 150 via the outlet portion 151. The separable portion 160b separated from the cylindrical part 160a is stopped by the inner surface of the outlet portion 151 at the tip end so as not to flow into the circular tube 2.

The sclerosing agent foam may be injected into a vein using a winged needle unit 60 shown in FIG. 18. The winged needle unit 60 includes a needle 61, a cylindrical connector 62, and a single wing 63 radially protruding from the peripheral surface of the connector 62. The needle 61 has a blade face 61a which faces upward when the wing 63 is held vertically to stand upright. With such a structure where the direction in which the wing 63 is protruding conforms with the orientation of the blade face 61a, the blade face 61a can have such an orientation that the needle 61 can be stuck into the skin when the operator holds the wing 63 upright. Thus, the wing 63 can be used as a tab for holding the needle unit 60 in the operation of sticking the needle 61 into the skin and makes the needling operation easier.

It should be noted that, in the examples of this embodiment described herein, the sclerosing agent is Polidocanol but may be an agent used in sclerotherapy other than Polidocanol.

One of the medical fluid container and the gas container may be a syringe while the other may be a bag. In this case, the syringe containing the prepared sclerosing agent foam is disengaged from the instrument 1, and the sclerosing agent foam is injected into a vein from the syringe.

Alternatively, the circular tube 2, the medical fluid syringe 100 and the air syringe 200 may be combined to form a device for varicose sclerotherapy. In this case, one of or both of the medical fluid syringe 100 and the air syringe 200 may be a bag. The device for varicose sclerotherapy may further include the coil spring 210. The device for varicose sclerotherapy may further include the winged needle unit 60.

The gas to be mixed with the sclerosing agent may be a gas other than air, such as carbon dioxide gas, or the like.

### Industrial Applicability

As described above, an instrument for varicose sclerotherapy according to the present invention can be used for mixing air with the sclerosing agent, such as Polidocanol, to produce foam of the sclerosing agent.

## Claims

1. An instrument for varicose sclerotherapy, comprising:
a medical fluid container connecting portion to which an opening of a medical fluid container for a sclerosing agent is to be connected;
a gas container connecting portion to which an opening of a gas container for a gas is to be connected; and
a mixing portion provided between the connecting portions, the mixing portion having a communication passageway for communication between the openings of the containers connected to the connecting portions, the communication passageway allowing mixture of the sclerosing agent and the air flowing therethrough,
wherein the mixing portion is deformable such that relative positions of the connecting portions can be changed.

2. The instrument of claim 1, wherein the mixing portion is deformable such that the medical fluid container and the gas container are positioned side by side.

3. The instrument of claim 1 or 2, wherein the mixing portion has a narrow part in which the communication passageway is narrower.

4. The instrument of any one of claims 1 to 3, wherein the communication passageway has a projection elevated toward the inside of the communication passageway.

5. The instrument of any one of claims 1 to 4, wherein the mixing portion is made of a resin.

6. The instrument of claim 5, wherein the mixing portion has a tubular part and is deformable such that the tubular part is bent.

7. The instrument of any one of claims 1 to 6, wherein:
the medical fluid container and the gas container are syringes; and
the medical fluid container connecting portion and the gas container connecting portion are designed such that tapered surfaces provided in external surfaces of the syringes at the tip ends fit in the connecting portions.

8. The instrument of any one of claims 1 to 7, wherein:
at least one of the medical fluid container and the gas container is a syringe with a rod inserted therein; and
the syringe is provided with an urging member for urging the rod in such a direction that the rod is extracted from the syringe.

9. The instrument of claim 8, wherein the urging member is a spring.

10. The instrument of any one of claims 1 to 6, wherein the medical fluid container and the gas container are bags.

11. The instrument of claim 10, wherein an opening of at least one of the medical fluid container and the gas container is detachably connected to the connecting portion.

12. A device for varicose sclerotherapy, comprising:
a medical fluid container containing a sclerosing agent;
a gas container containing a gas; and
an instrument connecting the medical fluid container and the gas container,
wherein the instrument includes a medical fluid container connecting portion to which an opening of the medical fluid container is connected, a gas container connecting portion to which an opening of the gas container is connected, and a mixing portion provided between the connecting portions, the mixing portion having a communication passageway for communication between the openings of the containers connected to the connecting portions, the communication passageway allowing mixture of the sclerosing agent and the air flowing therethrough, and
the mixing portion is deformable such that relative positions of the connecting portions can be changed.
